# EUROPEAN PATENT APPLICATION

(11) **EP 4 825 415 A1**
(43) Date of publication of application: **21.10.2026**
(21) Application number: 24902610.5
(22) Date of filing: 02.12.2024
(51) Int. Cl.: A61M 5/20

(54) **SPIRAL EXCITATION ASSEMBLY OF PREFILLED AUTOMATIC INJECTION PEN AND PREFILLED AUTOMATIC INJECTION PEN**

(30) Priority: 14.12.2023 CN 202311724367; 14.12.2023 CN 202311723803
(71) Applicant: Shanghai Innopac Medical Technology Co., Ltd., Shanghai 201605 (CN)
(72) Inventor: GUO, Rong, Shanghai 201605 (CN); WU, Ding, Shanghai 201605 (CN)
(74) Representative: karo IP
(86) International application number: PCT/CN2024/136046
(87) International publication number: WO 2025/124189

(57) **Abstract**

The present invention provides a screw-type activation assembly for a pre-filled auto-injector pen and a pre-filled auto-injector pen. The screw-type activation assembly for a pre-filled auto-injector pen comprises: a cavity body configured to convert rotational motion of a threaded push rod into axial forward movement; a nut rotatably mounted within the cavity body relative to the cavity body, wherein a torsion spring capable of driving the nut to rotate is provided between the nut and the cavity body; a threaded push rod threadedly connected within the nut and driven, upon forward movement caused by rotation of the nut, to drive a pre-filled syringe to move so as to complete automatic needle insertion; a clutch member sleeved outside the nut, capable of moving from an engagement position to an activation position under driving of an activation sleeve of the pre-filled auto-injector pen, wherein when at the engagement position, the clutch member engages with the nut to stop the nut, and when at the activation position, the nut is capable of rotating; and a clutch spring mounted between the clutch member and an end cap, and configured, after completion of injection, to drive the clutch member to move from the activation position into a limiting position to restrict rearward movement of the activation sleeve for needle protection, and to cause the clutch member to strike the nut to produce a sound.

## Description

### Technical Field

The present invention belongs to the field of auto-injection technology, and specifically relates to a screw-type activation assembly for a pre-filled auto-injector pen and a pre-filled auto-injector pen.

### Background Art

Pre-filled auto-injector pens currently on the market have the following drawbacks:
1. At present, in order to achieve the automatic injection function, this category of products employs electric motors and the like as the power source for the injection mechanism. Such a manner consumes energy, has a high cost of use, requires a complex control system, and requires a battery, which causes environmental pollution. Connection with various transmission mechanisms causes the product to be bulky overall, making it impossible to meet portability and ease-of-use requirements.
2. At present, in order to achieve multi-dimensional feedback, relevant products on the market mostly rely on electronic components to provide visual and auditory feedback, and the products still require battery power. If the product is used in an environment with strong electromagnetic interference, the product is unable to perform its intended functions. Electronic components are easily affected by the environment, are prone to aging, and are not suitable for long-term storage. It is difficult to ensure stability and reliability of the entire apparatus during use in complex environments.
3. At present, such products on the market involve a relatively large number of operating steps, often requiring at least three or more operating steps; risks of forgetting steps still exist when the patient uses the product periodically, resulting in poor patient compliance.
4. At present, after use of products on the market, the user is often required to insert a limiting pin or rotate a locking mechanism in order to achieve self-locking of the product, thereby preventing secondary use and accidental triggering. However, under certain specific circumstances, situations such as loss of limiting parts or the patient forgetting to perform the operation may occur, with the result that the product cannot ensure its self-locking after use. Moreover, such a design is contrary to human-factors engineering and does not conform to the design characteristics of this category of products.
5. In existing products, in order to achieve the automatic injection function, the trigger mechanism mostly adopts structures such as snap-fits, utilizing elastic deformation of components to complete processes such as triggering and locking. Such designs often require the snap-fits or like structures to be in a compressed-and-deformed state. Since such products are for single use, the components are usually injection-molded from polymer materials; placing snap-fit structures of the components in a compressed state is unfavorable for transportation and long-term storage; and being greatly affected by environment, such structures are prone to plastic deformation.

### Summary of the Invention

Aimed at the problems existing in the above background art, an object of the present invention is to provide a screw-type activation assembly for a pre-filled auto-injector pen and a pre-filled auto-injector pen which has reliable activation, has a reliable injection-completion reminder function, and is capable of achieving needle protection.

The technical solution adopted by the present invention is as follows:
A screw-type activation assembly for a pre-filled auto-injector pen, comprising:
a cavity body fixed on a housing of the pre-filled auto-injector pen and configured to convert rotational motion of a threaded push rod into axial forward movement;
a nut rotatably mounted within the cavity body relative to the cavity body, wherein a torsion spring capable of driving the nut to rotate is provided between the nut and the cavity body;
a threaded push rod threadedly connected within the nut and driven, upon forward movement caused by rotation of the nut, to drive a pre-filled syringe to move so as to complete automatic needle insertion;
a clutch member sleeved outside the nut, capable of moving from an engagement position to an activation position under driving of an activation sleeve of the pre-filled auto-injector pen, wherein when at the engagement position, the clutch member engages with the nut to stop the nut, and when at the activation position, the nut is capable of rotating;
an end cap sleeved and detachably connected to a rear end of the nut; and
a clutch spring mounted between the clutch member and the end cap, and configured, after completion of injection, to drive the clutch member to move from the activation position into a limiting position to restrict rearward movement of the activation sleeve for needle protection, and to cause the clutch member to strike the nut to produce a sound.

According to the present invention, after activation, forward movement of the threaded push rod is driven via rotation of the nut so as to drive movement of the pre-filled syringe to complete automatic needle insertion, which is more reliable compared with a form in which a spring directly drives the push rod. Meanwhile, the clutch member is incorporated to achieve activation and needle protection, which is more reliable than processes in which elastic deformation completes triggering and locking; moreover, the clutch member also cooperates with the nut to produce a sound upon injection completion, which is structurally simpler and more rational and provides smoother cooperation.

Further, the clutch member is a hollow cylindrical structure, an outer surface of the clutch member being symmetrically provided with protruding lugs for cooperating with the activation sleeve for driving or limiting.

Further, a first sliding slot for the lugs to move from the engagement position to the activation position, and a second sliding slot for the lugs to move from the activation position to the limiting position, are formed between a front end surface of the end cap and a rear end surface of the cavity body; the first sliding slot and the second sliding slot are in communication with each other; and the lugs are arranged to protrude from the first sliding slot and the second sliding slot. According to the present invention, the lugs are always in contact with the activation sleeve during the movement from the engagement position to the activation position.

Further, a front end surface of the end cap is symmetrically provided with right-angled trapezoidal grooves each having an outer-wide-inner-narrow shape; a rear end surface of the cavity body is symmetrically provided with oblique W-shaped grooves; an opening-end width of each right-angled trapezoidal groove is smaller than an opening-end width of each oblique W-shaped groove; an oblique direction of the right-angled trapezoidal grooves is consistent with an oblique direction of the oblique W-shaped grooves; and the first sliding slot and the second sliding slot in communication with each other are formed between the right-angled trapezoidal grooves and the oblique W-shaped grooves. According to the present invention, since the opening-end width of each right-angled trapezoidal groove is smaller than the opening-end width of each oblique W-shaped groove, when the lugs enter the limiting position from the activation position along the second sliding slot, the lugs are limited at the limiting position, thereby being capable of restricting rearward movement of the activation sleeve.

Further, engagement teeth are provided on both a front end surface of the clutch member and an end surface at a middle of the nut; when at the engagement position, the engagement teeth on the clutch member engage with the engagement teeth on the nut for stopping; and when entering the limiting position, the engagement teeth on the clutch member and the engagement teeth on the nut are capable of striking each other to produce a sound.

Further, the nut comprises a nut body of a hollow cylindrical structure, an interior of the nut body being provided with internal threads for cooperative threaded driving with the threaded push rod.

Further, a middle portion of the nut body is annularly provided with a shroud having an opening facing forward; a recessed cavity for accommodating the torsion spring is formed between the shroud and the nut body; and the engagement teeth on the nut are provided on a rear end surface of the shroud.

Further, a surface of the shroud is annularly provided with a raised rib; the cavity body is annularly provided, at a position opposite the raised rib, with a groove; and the raised rib is located within the groove to axially limit the nut.

Further, a rear end of the nut body is provided with an L-shaped positioning groove; and an interior of the end cap is provided with a protruding positioning member for cooperative detachable connection with the positioning groove.

Further, a front end surface of the cavity body and the threaded push rod are in flat-portion fit, thereby restricting the threaded push rod from rotating and allowing only axial movement.

A screw-activated pre-filled auto-injector pen, comprising:
a housing having a hollow structure;
a protective cap engaged with a head of the housing and capable, when pulled off, of driving an inner protective rubber sleeve of a pre-filled syringe to detach therewith;
an activation sleeve movably mounted within the housing, with a return spring for resetting being axially provided between the activation sleeve and the housing; a head of the activation sleeve being exposed outside the housing, and the activation sleeve being capable, when moving from an initial position to a limit position, of triggering a screw-type activation assembly;
a pre-filled syringe clamped on a PFS holder when pre-mounted, wherein during movement of the activation sleeve from the initial position to the limit position, an injection end of the pre-filled syringe is always concealed within a needle shield, and when the injection end of the pre-filled syringe is triggered, the pre-filled syringe is instantaneously displaced to extend beyond the activation sleeve to achieve automatic needle insertion;
a PFS holder fixedly mounted between the activation sleeve and the pre-filled syringe, configured to initially define an initial position of the pre-filled syringe and to provide support during automatic needle insertion of the pre-filled syringe;
wherein the above-described screw-type activation assembly for a pre-filled auto-injector pen is mounted within the housing.

Further, the activation sleeve comprises a sleeve body for protecting a needle; the sleeve body is provided with symmetrical extension arms; and a tail end of each of the extension arms is provided with an activation portion for cooperative activation with the lugs and a limiting portion for cooperative limiting with the lugs.

Compared with the prior art, the significant advantages of the present invention include:
1. After activation, the screw-type activation assembly drives forward movement of the threaded push rod via rotation of the nut so as to drive movement of the pre-filled syringe to complete automatic needle insertion, which is more reliable compared with a form in which a spring directly drives the push rod.
2. The clutch member is incorporated to achieve activation and needle protection, which is more reliable than processes in which elastic deformation completes triggering and locking; moreover, the clutch member also cooperates with the nut to produce a sound upon injection completion, which is structurally simpler and more rational and provides smoother cooperation.

### Brief Description of the Drawings

FIG. 1 is a schematic exploded structural view of the screw-type activation assembly of the present invention;
FIG. 2 is a schematic structural view of the cavity body of the screw-type activation assembly of the present invention;
FIG. 3 is a schematic structural view of the nut of the screw-type activation assembly of the present invention;
FIG. 4 is a schematic structural view of the clutch member of the screw-type activation assembly of the present invention;
FIG. 5 is a schematic structural view of the end cap of the screw-type activation assembly of the present invention;
FIG. 6 is a schematic cross-sectional structural view of the screw-type activation assembly of the present invention when the clutch member is at the engagement position;
FIG. 7 is a schematic cross-sectional structural view of the screw-type activation assembly of the present invention when the clutch member is at the activation position;
FIG. 8 is a schematic cross-sectional structural view of the screw-type activation assembly of the present invention when the clutch member is at the limiting position;
FIG. 9 is a schematic structural view of cooperation between the screw-type activation assembly of the present invention and the activation sleeve in an initial state;
FIG. 10 is a schematic structural view of the screw-type activation assembly of the present invention performing needle protection after activation.
FIG. 11 is a schematic exploded structural view of the pre-filled auto-injector pen of the present invention;
FIG. 12 is a schematic external structural view of the pre-filled auto-injector pen of the present invention;
FIG. 13 is a schematic structural view of the activation sleeve of the pre-filled auto-injector pen of the present invention;
FIG. 14 is a schematic cross-sectional structural view of the pre-filled auto-injector pen of the present invention when the clutch member is at the engagement position;
FIG. 15 is a schematic cross-sectional structural view of the pre-filled auto-injector pen of the present invention when the clutch member is at the activation position;
FIG. 16 is a schematic cross-sectional structural view of the pre-filled auto-injector pen of the present invention when the clutch member is at the limiting position.

Reference signs:
1 - protective cap; 1a - protective cap catch; 2 - housing; 21 - viewing window; 3 - activation sleeve; 31 - sleeve body; 32 - extension arm; 33 - activation portion; 34 - limiting portion; 35 - slot hole; 36 - protection catch; 4 - pre-filled syringe; 41 - inner protective rubber sleeve; 42 - protruding tail end; 5 - threaded push rod; 6 - cavity body; 61 - oblique W-shaped groove; 62 - groove; 7 - torsion spring; 8 - clutch spring; 9 - nut; 91 - nut body; 92 - shroud; 93 - recessed cavity; 94 - raised rib; 95 - positioning groove; 10 - clutch member; 101 - lug; A - engagement position; B - activation position; C - limiting position; 11 - end cap; 111 - right-angled trapezoidal groove; 112 - positioning member; 12 - PFS holder; 121 - cantilever; 122 - outer catch; 123 - inner catch; 124 - stepped surface; 13 - engagement teeth; 14 - first sliding slot; 15 - second sliding slot.

### Detailed Description of Embodiments

The present invention will be further described below in conjunction with specific embodiments, but the present invention is not limited to these specific embodiments. Those skilled in the art should recognize that the present invention encompasses all alternative solutions, modifications, and equivalents that may be included within the scope of the claims.

In the description of the present invention, it should be understood that the terms "center", "longitudinal", "transverse", "length", "width", "thickness", "upper", "lower", "front", "rear", "left", "right", "vertical", "horizontal", "top", "bottom", "inner", "clockwise", "counterclockwise" and the like indicate orientations or positional relationships based on the orientations or positional relationships shown in the drawings, and are merely for facilitating description of the present invention and simplifying description, rather than indicating or implying that the referred device or element must have a specific orientation or be constructed and operated in a specific orientation, and therefore cannot be construed as limiting the present invention. Furthermore, the terms "first" and "second" are used for descriptive purposes only and cannot be construed as indicating or implying relative importance or implicitly indicating the number of the technical features indicated. Thus, features defined with "first" or "second" may explicitly or implicitly include one or more such features. In the description of the present invention, unless otherwise specified, "a plurality of" means two or more, unless otherwise expressly defined.

In the present invention, unless otherwise expressly stipulated and defined, the terms "mount", "couple", "connect", "fix" and the like should be understood in a broad sense. For example, they may refer to a fixed connection, a detachable connection, or an integral connection; may be a mechanical connection or an electrical connection; may be a direct connection, or may be an indirect connection through an intermediate medium, or may be a communication between interiors of two elements. For those of ordinary skill in the art, the specific meanings of the above terms in the present invention can be understood according to specific circumstances.

In the present invention, unless otherwise expressly stipulated and defined, a first feature being "above" or "below" a second feature may include the first and second features being in direct contact, or may include the first and second features being not in direct contact but in contact through another feature therebetween. Moreover, the first feature being "above", "over" and "on top of" the second feature includes the first feature being directly above and obliquely above the second feature, or merely indicates that the horizontal height of the first feature is higher than that of the second feature. The first feature being "below", "under" and "beneath" the second feature includes the first feature being directly below and obliquely below the second feature, or merely indicates that the horizontal height of the first feature is less than that of the second feature.

### Embodiment 1

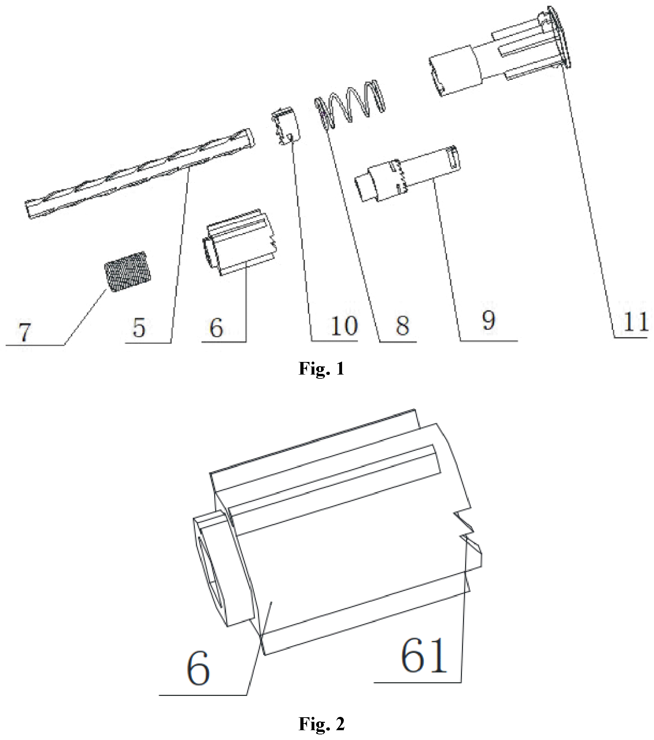

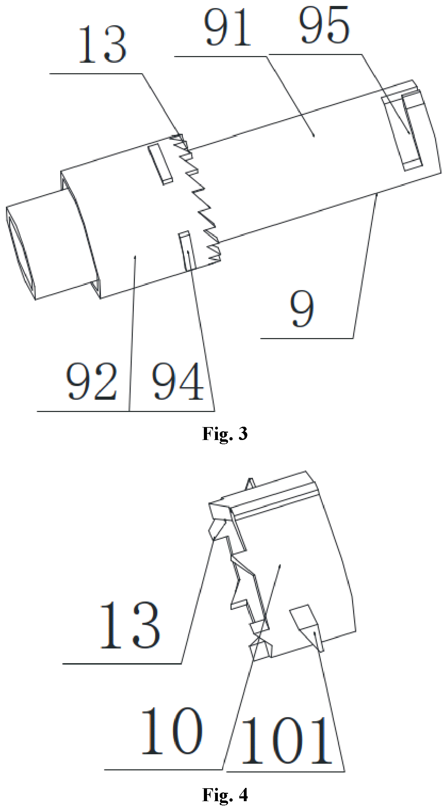

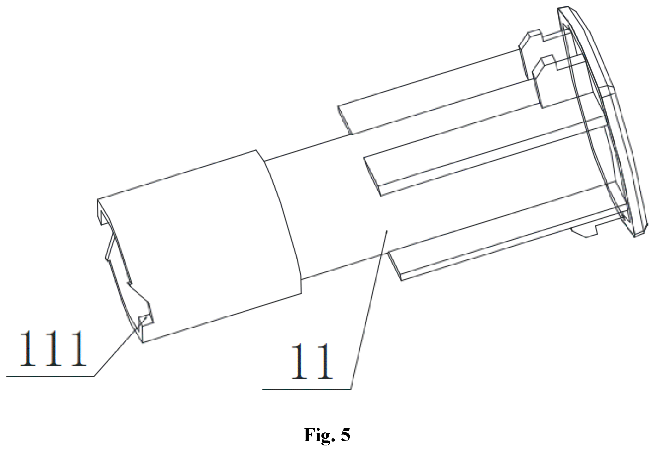

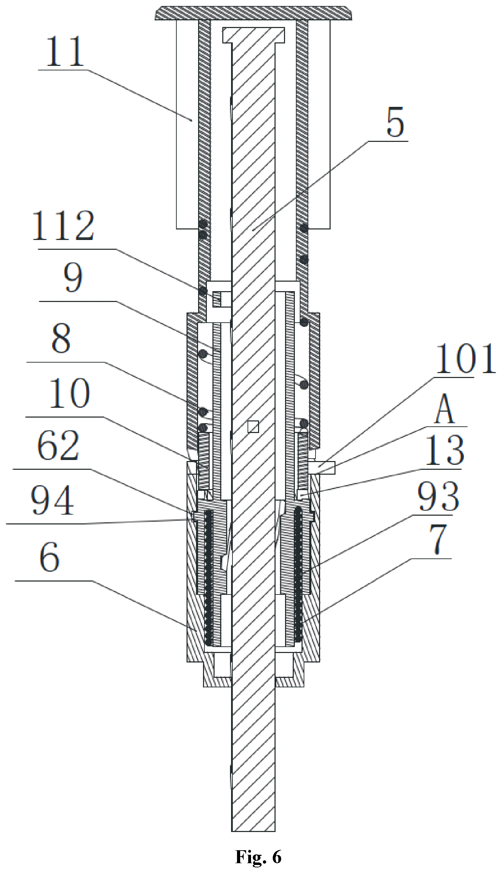

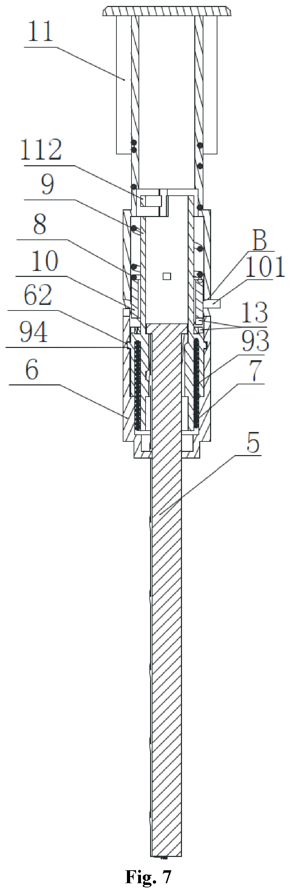

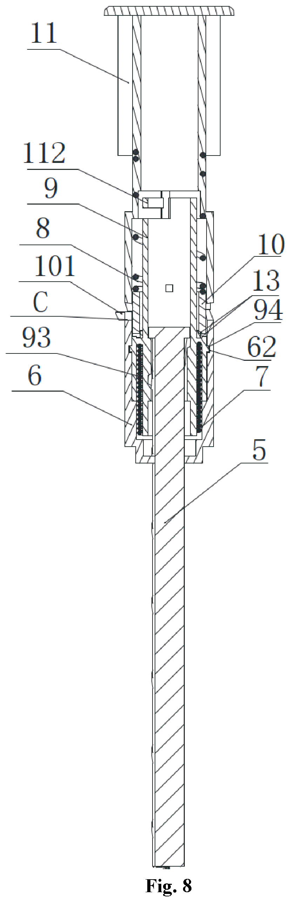

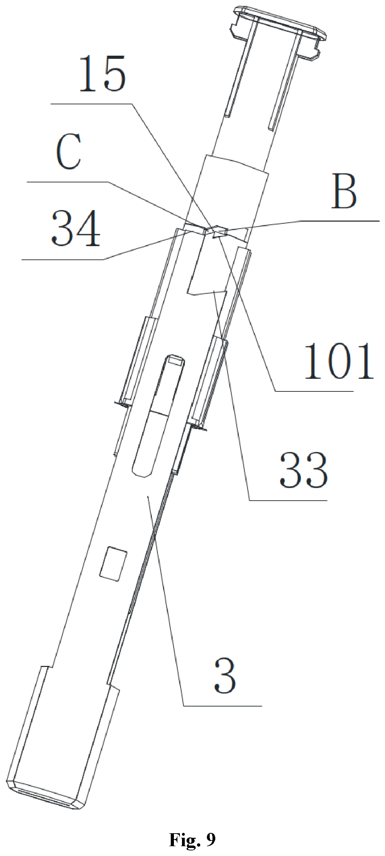

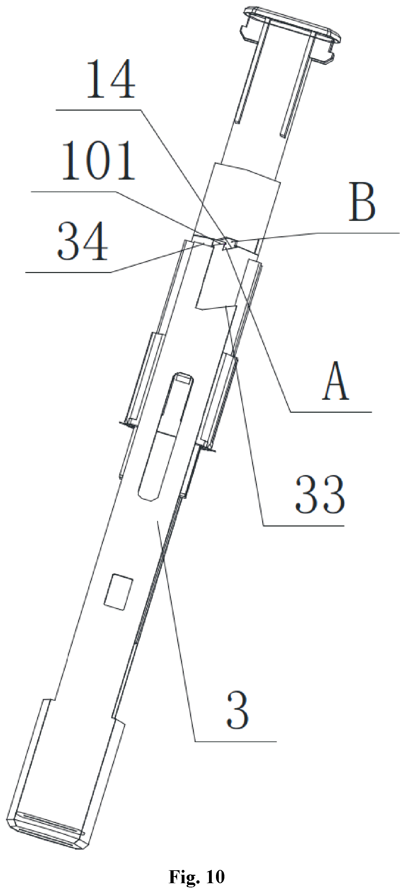

Referring to FIGS. 1-10, the present embodiment provides a screw-type activation assembly for a pre-filled auto-injector pen, comprising:
a cavity body 6 fixed on a housing 2 of the pre-filled auto-injector pen and configured to convert rotational motion of a threaded push rod 5 into axial forward movement;
a nut 9 rotatably mounted within the cavity body 6 relative to the cavity body 6, wherein a torsion spring 7 capable of driving the nut 9 to rotate is provided between the nut 9 and the cavity body 6;
the threaded push rod 5, threadedly connected within the nut 9 and driven, upon forward movement caused by rotation of the nut 9, to drive a pre-filled syringe 4 to move so as to complete automatic needle insertion;
a clutch member 10 sleeved outside the nut 9, capable of moving from an engagement position A to an activation position B under driving of an activation sleeve 3 of the pre-filled auto-injector pen, wherein when at the engagement position A, the clutch member 10 engages with the nut 9 to stop the nut 9, and when at the activation position B, the nut 9 is capable of rotating;
an end cap 11 sleeved and detachably connected to a rear end of the nut 9; and
a clutch spring 8 mounted between the clutch member 10 and the end cap 11, and configured, after completion of injection, to drive the clutch member 10 to move from the activation position B into a limiting position C to restrict rearward movement of the activation sleeve 3 for needle protection, and to cause the clutch member 10 to strike the nut 9 to produce a sound.

According to the present invention, after activation, forward movement of the threaded push rod 5 is driven via rotation of the nut 9 so as to drive movement of the pre-filled syringe 4 to complete automatic needle insertion, which is more reliable compared with a form in which a spring directly drives the push rod. Meanwhile, the clutch member 10 is incorporated to achieve activation and needle protection, which is more reliable than processes in which elastic deformation completes triggering and locking; moreover, the clutch member 10 also cooperates with the nut 9 to produce a sound upon injection completion, which is structurally simpler and more rational and provides smoother cooperation.

The clutch member 10 in the present embodiment is a hollow cylindrical structure, an outer surface of the clutch member 10 being symmetrically provided with protruding lugs 101 for cooperating with the activation sleeve 3 for driving or limiting. A first sliding slot 14 for the lugs 101 to move from the engagement position A to the activation position B, and a second sliding slot 15 for the lugs 101 to move from the activation position B to the limiting position C, are formed between a front end surface of the end cap 11 and a rear end surface of the cavity body 6; the first sliding slot 14 and the second sliding slot 15 are in communication with each other; and the lugs 101 are arranged to protrude from the first sliding slot 14 and the second sliding slot 15. According to the present invention, the lugs 101 are always in contact with the activation sleeve 3 during the movement from the engagement position A to the activation position B. Specifically, a front end surface of the end cap 11 is symmetrically provided with right-angled trapezoidal grooves 111 each having an outer-wide-inner-narrow shape; a rear end surface of the cavity body 6 is symmetrically provided with oblique W-shaped grooves 61; an opening-end width of each right-angled trapezoidal groove 111 is smaller than an opening-end width of each oblique W-shaped groove 61; an oblique direction of the right-angled trapezoidal grooves 111 is consistent with an oblique direction of the oblique W-shaped grooves 61; and the first sliding slot 14 and the second sliding slot 15 in communication with each other are formed between the right-angled trapezoidal grooves 111 and the oblique W-shaped grooves 61. According to the present invention, since the opening-end width of each right-angled trapezoidal groove 111 is smaller than the opening-end width of each oblique W-shaped groove 61, when the lugs 101 enter the limiting position C from the activation position B along the second sliding slot 15, the lugs 101 are limited at the limiting position C, thereby being capable of restricting rearward movement of the activation sleeve 3. The first sliding slot 14 and the second sliding slot 15 are formed by cooperation between the end cap 11 and the cavity body 6, which facilitates mounting and dismounting of the clutch member 10. Of course, the first sliding slot 14 and the second sliding slot 15 may also be directly provided on one of the components.

In the present embodiment, engagement teeth 13 are provided on both a front end surface of the clutch member 10 and an end surface at a middle of the nut 9; when at the engagement position A, the engagement teeth 13 on the clutch member 10 engage with the engagement teeth 13 on the nut 9 for stopping. When entering the limiting position C, the engagement teeth 13 on the clutch member 10 and the engagement teeth 13 on the nut 9 are capable of striking each other to produce a sound. The engagement teeth 13 on the nut 9 are continuously arranged, and the engagement teeth 13 on the clutch member 10 are spaced apart.

The nut 9 in the present embodiment comprises a nut body 91 of a hollow cylindrical structure, an interior of the nut body 91 being provided with internal threads for cooperative threaded driving with the threaded push rod 5. A middle portion of the nut body 91 is annularly provided with a shroud 92 having an opening facing forward; a recessed cavity 93 for accommodating the torsion spring 7 is formed between the shroud 92 and the nut body 91; and the engagement teeth 13 on the nut 9 are provided on a rear end surface of the shroud 92. A surface of the shroud 92 is annularly provided with a raised rib 94; the cavity body 6 is annularly provided, at a position opposite the raised rib 94, with a groove 62; and the raised rib 94 is located within the groove 62 to axially limit the nut 9, such that the nut 9 can only rotate relative to the cavity body 6. A rear end of the nut body 91 is provided with an L-shaped positioning groove 95; and an interior of the end cap 11 is provided with a protruding positioning member 112 for cooperative detachable connection with the positioning groove 95.

In the present embodiment, a front end surface of the cavity body 6 and the threaded push rod 5 are in flat-portion fit; that is, a through-hole at the front end of the cavity body 6 is a flat hole, the threaded push rod 5 is of a flat structure, and external threads are provided on a curved surface thereof, thereby restricting the threaded push rod 5 from rotating and allowing only axial movement.

In an initial state of the present invention, the clutch member 10 engages with the nut 9, and at this time the threaded push rod 5 cannot be pushed. During use, when the activation sleeve 3 moves axially, an activation portion 33 thereof contacts the lugs 101 of the clutch member 10 and drives the lugs 101 to move from the engagement position A to the activation position B, thereby driving the clutch member 10 to leave the engagement position; at this time, the nut 9 rotates under driving of the torsion spring 7, thereby driving the threaded push rod 5 to rotate; under the restriction of the cavity body 6, the rotation of the threaded push rod 5 is converted into axial forward movement, such that the pre-filled syringe of the pre-filled auto-injector pen can be activated to move for automatic needle insertion. After completion of injection, the activation sleeve 3 springs back, the activation portion thereof leaves the lugs 101, and the lugs 101 move from the activation position B to the limiting position C under action of the clutch spring 8; at this time, the lugs 101 contact a limiting portion 34 of the activation sleeve 3, thereby preventing the activation sleeve 3 from again being moved axially rearward, thus achieving needle protection, and causing the engagement teeth 13 of the clutch member 10 and the engagement teeth 13 of the nut 9 to strike each other to produce a sound.

According to the present invention, after activation, forward movement of the threaded push rod 5 is driven via rotation of the nut 9 so as to drive movement of the pre-filled syringe to complete automatic needle insertion, which is more reliable compared with a form in which a spring directly drives the push rod. The clutch member 10 is incorporated to achieve activation and needle protection, which is more reliable than processes in which elastic deformation completes triggering and locking; moreover, the clutch member 10 also cooperates with the nut 9 to produce a sound upon injection completion, which is structurally simpler and more rational and provides smoother cooperation.

### Embodiment 2

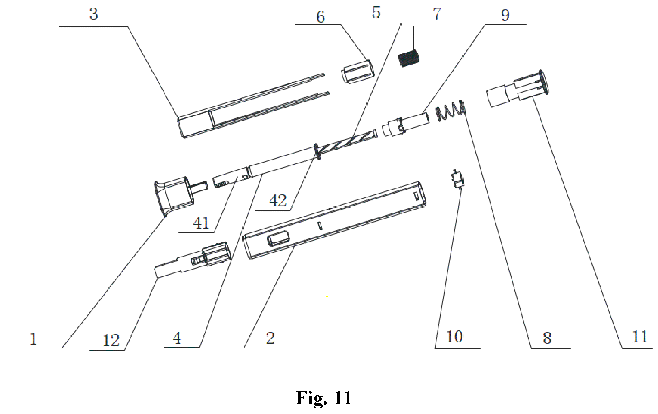

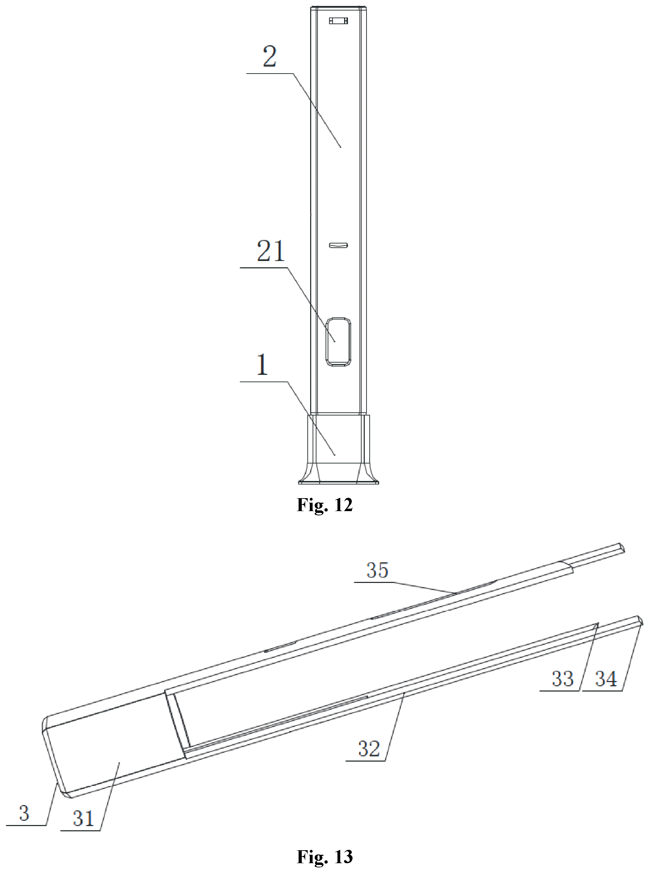

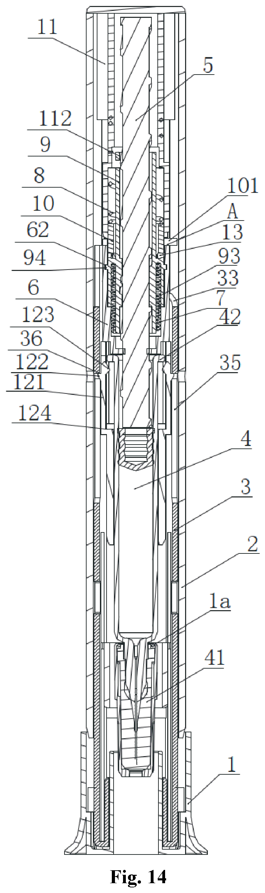

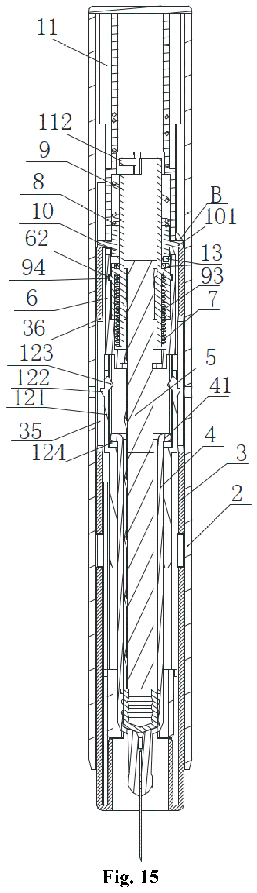

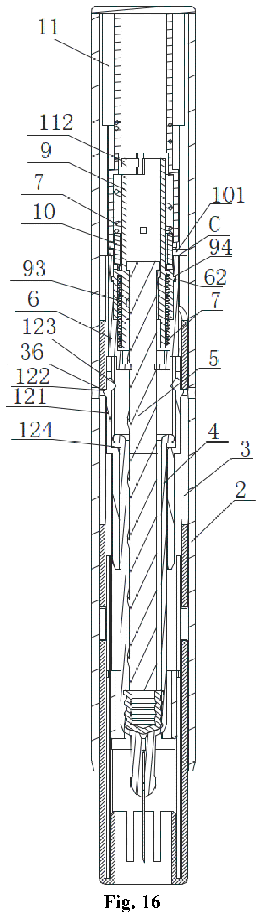

Referring to FIGS. 11-16, the present embodiment provides a screw-activated pre-filled auto-injector pen, comprising a protective cap 1, a housing 2, an activation sleeve 3, a pre-filled syringe 4, a threaded push rod 5, a cavity body 6, a torsion spring 7, a clutch spring 8, a nut 9, a clutch member 10, an end cap 11, and a PFS holder 12, wherein the threaded push rod 5, the cavity body 6, the torsion spring 7, the clutch spring 8, the nut 9, the clutch member 10, and the end cap 11 constitute the screw-type activation assembly, and the activation assembly is mounted within the housing 2.

The protective cap 1 in the present embodiment is engaged with a head of the housing 2 and, when pulled off, is capable of driving an inner protective rubber sleeve 41 of the pre-filled syringe 4 to detach therewith. Specifically, the protective cap 1 is provided with symmetrically arranged protective cap catches 1a, and the protective cap catches 1a are initially snap-connected with the inner protective rubber sleeve 41 of the pre-filled syringe 4. The inner protective rubber sleeve 41 of the pre-filled syringe 4 disengages from the pre-filled syringe 4 when the protective cap 1 is pulled off, thereby exposing the needle.

The housing 2 in the present embodiment has a hollow structure; the housing 2 is provided with a viewing window 21 for inspecting a state of medicinal liquid, whether a position of the pre-filled syringe is correct, and an infusion condition after completion of injection.

The activation sleeve 3 in the present embodiment is movably mounted within the housing 2, with a return spring for resetting being axially provided between the activation sleeve 3 and the housing 2; a head of the activation sleeve 3 is exposed outside the housing 2, and the activation sleeve 3 is capable, when moving from an initial position to a limit position, of triggering the screw-type activation assembly. Specifically, the activation sleeve 3 comprises a sleeve body 31 for protecting the needle; the sleeve body 31 is provided with symmetrical extension arms 32; and a tail end of each of the extension arms 32 is provided with an activation portion 33 for cooperative activation with the lugs 101 on the clutch member 10 and a limiting portion 34 for cooperative limiting with the lugs 101. Each of the extension arms 32 is provided with a slot hole 35 for movement of a cantilever 121 of the PFS holder 12; and an inner side of each of the extension arms 32 is provided, at one end of the slot hole 35, with a protection catch 36 protruding radially for defining an initial position of the cantilever 121 of the PFS holder 12.

The pre-filled syringe 4 in the present embodiment is clamped on the PFS holder 12 when pre-mounted; during movement of the activation sleeve 3 from the initial position to the limit position, an injection end of the pre-filled syringe 4 is always concealed within a needle shield; when the injection end of the pre-filled syringe 4 is triggered, the pre-filled syringe 4 is instantaneously displaced to extend beyond the activation sleeve 3 to achieve automatic needle insertion. Specifically, the position at which the pre-filled syringe 4 is clamped on the cantilever 121 of the PFS holder 12 when pre-mounted is the initial position of the pre-filled syringe 4; during activation, the activation sleeve 3 moves from the initial position to the limit position; when the activation sleeve 3 is at the limit position, the injection end of the pre-filled syringe 4 is concealed within the activation sleeve 3; when the injection end of the pre-filled syringe 4 is triggered, the pre-filled syringe 4 is instantaneously displaced, moving from the initial position to a stepped surface 124 on the PFS holder 12 and projecting beyond the activation sleeve 3 to achieve automatic needle insertion; the injection end is a needle structure, initially provided with the inner protective rubber sleeve 41 for protecting the injection end; a liquid is pre-filled within the pre-filled syringe 4 and is sealed by a rubber plunger; and a protruding tail end 42 of the pre-filled syringe 4 is protrudingly arranged, can be defined by the PFS holder 12 at the initial position, and cooperates with the stepped surface 124 on the PFS holder 12 to limit a movement distance.

The threaded push rod 5 in the present embodiment is threadedly connected within the nut 9 and is driven, upon forward movement caused by rotation of the nut 9, to drive the pre-filled syringe 4 to move so as to complete automatic needle insertion. The threaded push rod 5 is of a flat structure, and external threads are provided on a curved surface thereof to threadedly rotate in cooperation with the internal threads inside the nut 9.

The cavity body 6 in the present embodiment is a hollow structure, fixed on the housing 2, and is configured to convert rotational motion of the threaded push rod 5 into axial forward movement.

Specifically, a front end surface of the cavity body 6 and the threaded push rod 5 are in flat-portion fit; that is, a through-hole at the front end of the cavity body 6 is a flat hole, thereby restricting the threaded push rod 5 from rotating and allowing only axial movement. The cavity body 6 is annularly provided, at a position opposite the raised rib 94 on the nut 9, with a groove 62; and the raised rib 94 is located within the groove 62 to axially limit the nut 9, such that the nut 9 can only rotate relative to the cavity body 6.

The clutch spring 8 in the present embodiment is mounted between the clutch member 10 and the end cap 11, and is configured, after completion of injection, to drive the clutch member 10 to move from the activation position B into the limiting position C to restrict rearward movement of the activation sleeve 3 for needle protection, and to cause the clutch member 10 to strike the nut 9 to produce a sound.

The nut 9 in the present embodiment is rotatably mounted within the cavity body 6 relative to the cavity body 6, and the torsion spring 7 capable of driving the nut 9 to rotate is provided between the nut 9 and the cavity body 6. The nut 9 in the present embodiment comprises a nut body 91 of a hollow cylindrical structure, an interior of the nut body 91 being provided with internal threads for cooperative threaded driving with the threaded push rod 5. A middle portion of the nut body 91 is annularly provided with a shroud 92 having an opening facing forward; a recessed cavity 93 for accommodating the torsion spring 7 is formed between the shroud 92 and the nut body 91; and the engagement teeth 13 on the nut 9 are provided on a rear end surface of the shroud 92. A surface of the shroud 92 is annularly provided with a raised rib 94; the raised rib 94 is located within the groove 62 to axially limit the nut 9, such that the nut 9 can only rotate relative to the cavity body 6. A rear end of the nut body 91 is provided with an L-shaped positioning groove 95 for connecting and fixing the end cap 11. According to the present invention, after activation, forward movement of the threaded push rod 5 is driven via rotation of the nut 9 so as to drive movement of the pre-filled syringe to complete automatic needle insertion, which is more reliable compared with a form in which a spring directly drives the push rod.

The clutch member 10 in the present embodiment is sleeved outside the nut 9, is capable of moving from the engagement position A to the activation position B under driving of the activation sleeve 3, engages with the nut 9 to stop the nut 9 when at the engagement position A, and allows the nut 9 to rotate when at the activation position B. Specifically, the clutch member 10 is a hollow cylindrical structure, an outer surface of the clutch member 10 being symmetrically provided with protruding lugs 101 for cooperating with the activation sleeve 3 for driving or limiting. Engagement teeth 13 are provided on both a front end surface of the clutch member 10 and the end surface at the middle of the nut 9; when at the engagement position A, the engagement teeth 13 on the clutch member 10 engage with the engagement teeth 13 on the nut 9 for stopping. When entering the limiting position C, the engagement teeth 13 on the clutch member 10 and the engagement teeth 13 on the nut 9 are capable of striking each other to produce a sound. The engagement teeth 13 on the nut 9 are continuously arranged, and the engagement teeth 13 on the clutch member 10 are spaced apart. The clutch member 10 is incorporated to achieve activation and needle protection, which is more reliable than processes in which elastic deformation completes triggering and locking; moreover, the clutch member 10 also cooperates with the nut 9 to produce a sound upon injection completion, which is structurally simpler and more rational and provides smoother cooperation.

The end cap 11 in the present embodiment is sleeved and detachably connected to a rear end of the nut 9; an interior of the end cap 11 is provided with the protruding positioning member 112 for cooperative detachable connection with the positioning groove 95. A first sliding slot 14 for the lugs 101 to move from the engagement position A to the activation position B, and a second sliding slot 15 for the lugs 101 to move from the activation position B to the limiting position C, are formed between a front end surface of the end cap 11 and a rear end surface of the cavity body 6; the first sliding slot 14 and the second sliding slot 15 are in communication with each other; and the lugs 101 are arranged to protrude from the first sliding slot 14 and the second sliding slot 15. According to the present invention, the lugs 101 are always in contact with the activation sleeve 3 during the movement from the engagement position A to the activation position B. Specifically, the front end surface of the end cap 11 is symmetrically provided with right-angled trapezoidal grooves 111 each having an outer-wide-inner-narrow shape; the rear end surface of the cavity body 6 is symmetrically provided with oblique W-shaped grooves 61; an opening-end width of each right-angled trapezoidal groove 111 is smaller than an opening-end width of each oblique W-shaped groove 61; an oblique direction of the right-angled trapezoidal grooves 111 is consistent with an oblique direction of the oblique W-shaped grooves 61; and the first sliding slot 14 and the second sliding slot 15 in communication with each other are formed between the right-angled trapezoidal grooves 111 and the oblique W-shaped grooves 61. According to the present invention, since the opening-end width of each right-angled trapezoidal groove 111 is smaller than the opening-end width of each oblique W-shaped groove 61, when the lugs 101 enter the limiting position C from the activation position B along the second sliding slot 15, the lugs 101 are limited at the limiting position C, thereby being capable of restricting rearward movement of the activation sleeve 3. The first sliding slot 14 and the second sliding slot 15 are formed by cooperation between the end cap 11 and the cavity body 6, which facilitates mounting and dismounting of the clutch member 10. Of course, the first sliding slot 14 and the second sliding slot 15 may also be directly provided on one of the components.

The PFS holder 12 in the present embodiment is fixedly mounted between the activation sleeve 3 and the pre-filled syringe 4, initially defines the initial position of the pre-filled syringe 4 and is capable of providing support during automatic needle insertion of the pre-filled syringe 4. The PFS holder 12 is symmetrically provided with cantilevers 121; the cantilevers 121 are arranged corresponding to the slot holes 35 of the activation sleeve 3; an outer side of an end portion of each of the cantilevers 121 is provided with an outer catch 122 for initially cooperating with the protection catch 36 of the activation sleeve 3 to define a position of the cantilever 121; and an inner side of the end portion of each of the cantilevers 121 is provided with an inner catch 123 for initially cooperating with the protruding tail end 42 of the pre-filled syringe 4 to support the pre-filled syringe 4. An inner wall of the PFS holder 12 is provided with the stepped surface 124 for limiting movement of the pre-filled syringe 4; and the inner wall of the PFS holder 12 is axially provided, from the stepped surface 124 to a tail end thereof, with vertical ribs for providing support during automatic needle insertion of the pre-filled syringe 4. After the outer catch 122 of the cantilever 121 of the PFS holder 12 disengages from the protection catch 36, the cantilever 121 can deform within the slot hole 35, such that the pre-filled syringe 4 breaks through the inner catch 123 to move downward to the stepped surface 124 under action of the threaded push rod 5. A silicone gasket may be arranged on the stepped surface 124 for protecting the protruding tail end 42 of the pre-filled syringe 4 to prevent breakage during movement.

During assembly of the screw-type activation assembly of the present invention, the threaded push rod 5 is mounted into the nut 9 to constitute a nut assembly; the nut assembly and the torsion spring 7 are mounted into the cavity body 6, achieving a circumferential connection via the raised rib 94 and the groove 62; the clutch member 10 and the clutch spring 8 are sequentially sleeved outside the nut 9; and finally the end cap 11 is fixed at the rear end of the nut 9.

During assembly of the front portion of the pre-filled auto-injector pen, the return spring is mounted into the activation sleeve 3; then the activation sleeve 3 is mounted into the housing 2; the protective cap 1 is then mounted into the housing 2 to cover the activation sleeve 3; the PFS holder 12 is then mounted into the housing 2 to cooperatively position with the activation sleeve 3, at which time the cantilever 121 of the PFS holder 12 is located within the slot hole 35 of the activation sleeve 3, and the outer catch 122 is initially cooperatively positioned with the protection catch 36; the pre-filled syringe 4 is mounted into the PFS holder 12, at which time the inner catch 123 cooperates with the protruding tail end 42 for limiting. Finally, the assembled screw-type activation assembly is mounted into the housing 2 and is fixedly mounted by engagement of the cavity body 6 with the housing 2.

In an initial state of the present invention, the clutch member 10 engages with the nut 9, and at this time the threaded push rod 5 cannot be pushed. During use of the present invention, the protective cap 1 is pulled off to detach the inner protective rubber sleeve 41 from the pre-filled syringe 4. The activation sleeve 3 is pressed down, the activation sleeve 3 moves rearward, and when the activation portion 33 of the activation sleeve 3 contacts the lugs 101 of the clutch member 10, it drives the lugs 101 to move from the engagement position A to the activation position B, thereby driving the clutch member 10 to leave the engagement position; at this time, the nut 9 rotates under driving of the torsion spring 7, thereby driving the threaded push rod 5 to rotate; under the restriction of the cavity body 6, the rotation of the threaded push rod 5 is converted into axial forward movement, such that the pre-filled syringe of the pre-filled auto-injector pen can be activated to move for automatic needle insertion. After completion of injection, the activation sleeve 3 springs back, and the activation portion 33 thereof leaves the lugs 101; the lugs 101 move from the activation position B to the limiting position C under action of the clutch spring 8; at this time, the lugs 101 contact the limiting portion 34 of the activation sleeve 3, thereby preventing the activation sleeve 3 from again being moved axially rearward, thus achieving needle protection, and causing the engagement teeth 13 of the clutch member 10 and the engagement teeth 13 of the nut 9 to strike each other to produce a sound.

According to the present invention, after activation, forward movement of the threaded push rod 5 is driven via rotation of the nut 9 so as to drive movement of the pre-filled syringe to complete automatic needle insertion, which is more reliable compared with a form in which a spring directly drives the push rod. The clutch member 10 is incorporated to achieve activation and needle protection, which is more reliable than processes in which elastic deformation completes triggering and locking; moreover, the clutch member 10 also cooperates with the nut 9 to produce a sound upon injection completion, which is structurally simpler and more rational and provides smoother cooperation.

## Claims

1. A screw-type activation assembly for a pre-filled auto-injector pen, comprising:
a cavity body (6) fixed on a housing (2) and configured to convert rotational motion of a threaded push rod (5) into axial forward movement;
a nut (9) rotatably mounted within the cavity body (6) relative to the cavity body (6), wherein a torsion spring (7) capable of driving the nut (9) to rotate is provided between the nut (9) and the cavity body (6);
the threaded push rod (5), threadedly connected within the nut (9) and driven, upon forward movement caused by rotation of the nut (9), to drive a pre-filled syringe (4) to move so as to complete automatic needle insertion;
a clutch member (10) sleeved outside the nut (9), capable of moving from an engagement position (A) to an activation position (B) under driving of an activation sleeve (3), wherein when at the engagement position (A), the clutch member (10) engages with the nut (9) to stop the nut (9), and when at the activation position (B), the nut (9) is capable of rotating;
an end cap (11) sleeved and detachably connected to a rear end of the nut (9); and
a clutch spring (8) mounted between the clutch member (10) and the end cap (11), and configured, after completion of injection, to drive the clutch member (10) to move from the activation position (B) into a limiting position (C) to restrict rearward movement of the activation sleeve (3) for needle protection, and to cause the clutch member (10) to strike the nut (9) to produce a sound.

2. The screw-type activation assembly for a pre-filled auto-injector pen according to claim 1, wherein the clutch member (10) is a hollow cylindrical structure, and an outer surface of the clutch member (10) is symmetrically provided with protruding lugs (101) for cooperating with the activation sleeve (3) for driving or limiting.

3. The screw-type activation assembly for a pre-filled auto-injector pen according to claim 2, wherein a first sliding slot (14) for the lugs (101) to move from the engagement position (A) to the activation position (B), and a second sliding slot (15) for the lugs (101) to move from the activation position (B) to the limiting position (C), are formed between a front end surface of the end cap (11) and a rear end surface of the cavity body (6); the first sliding slot (14) and the second sliding slot (15) are in communication with each other; and the lugs (101) are arranged to protrude from the first sliding slot (14) and the second sliding slot (15).

4. The screw-type activation assembly for a pre-filled auto-injector pen according to claim 3, wherein a front end surface of the end cap (11) is symmetrically provided with right-angled trapezoidal grooves (111) each having an outer-wide-inner-narrow shape; a rear end surface of the cavity body (6) is symmetrically provided with oblique W-shaped grooves (61); an opening-end width of each right-angled trapezoidal groove (111) is smaller than an opening-end width of each oblique W-shaped groove (61); an oblique direction of the right-angled trapezoidal grooves (111) is consistent with an oblique direction of the oblique W-shaped grooves (61); and the first sliding slot (14) and the second sliding slot (15), in communication with each other, are formed between the right-angled trapezoidal grooves (111) and the oblique W-shaped grooves (61).

5. The screw-type activation assembly for a pre-filled auto-injector pen according to claim 1, wherein engagement teeth (13) are provided on both a front end surface of the clutch member (10) and an end surface at a middle of the nut (9); when at the engagement position (A), the engagement teeth (13) on the clutch member (10) engage with the engagement teeth (13) on the nut (9) for stopping; and when entering the limiting position (C), the engagement teeth (13) on the clutch member (10) and the engagement teeth (13) on the nut (9) are capable of striking each other to produce a sound.

6. The screw-type activation assembly for a pre-filled auto-injector pen according to claim 1, wherein the nut (9) comprises a nut body (91) of a hollow cylindrical structure; an interior of the nut body (91) is provided with internal threads for cooperative threaded driving with the threaded push rod (5); a middle portion of the nut body (91) is annularly provided with a shroud (92) having an opening facing forward; a recessed cavity (93) for accommodating the torsion spring (7) is formed between the shroud (92) and the nut body (91); and the engagement teeth (13) on the nut (9) are provided on a rear end surface of the shroud (92).

7. The screw-type activation assembly for a pre-filled auto-injector pen according to claim 6, wherein a surface of the shroud (92) is annularly provided with a raised rib (94); the cavity body (6) is annularly provided, at a position opposite the raised rib (94), with a groove (62); and the raised rib (94) is located within the groove (62) to axially limit the nut (9).

8. The screw-type activation assembly for a pre-filled auto-injector pen according to claim 6, wherein a rear end of the nut body (91) is provided with an L-shaped positioning groove (95); and an interior of the end cap (11) is provided with a protruding positioning member (112) for cooperative detachable connection with the positioning groove (95).

9. The screw-type activation assembly for a pre-filled auto-injector pen according to claim 1, wherein a front end surface of the cavity body (6) and the threaded push rod (5) are in flat-portion fit.

10. A screw-activated pre-filled auto-injector pen, comprising:
a housing (2) having a hollow structure;
a protective cap (1) engaged with a head of the housing (2) and capable, when pulled off, of driving an inner protective rubber sleeve (41) of a pre-filled syringe (4) to detach therewith;
an activation sleeve (3) movably mounted within the housing (2), with a return spring for resetting being axially provided between the activation sleeve (3) and the housing (2); a head of the activation sleeve (3) being exposed outside the housing (2), and the activation sleeve (3) being capable, when moving from an initial position to a limit position, of triggering a screw-type activation assembly;
the pre-filled syringe (4), clamped on a PFS holder (12) when pre-mounted, wherein during movement of the activation sleeve (3) from the initial position to the limit position, an injection end of the pre-filled syringe (4) is always concealed within a needle shield, and when the injection end of the pre-filled syringe (4) is triggered, the pre-filled syringe (4) is instantaneously displaced to extend beyond the activation sleeve (3) to achieve automatic needle insertion;
the PFS holder (12), fixedly mounted between the activation sleeve (3) and the pre-filled syringe (4), configured to initially define an initial position of the pre-filled syringe (4) and to provide support during automatic needle insertion of the pre-filled syringe (4);
wherein the screw-type activation assembly for a pre-filled auto-injector pen according to any one of claims 1 to 9 is mounted within the housing (2).

11. The screw-activated pre-filled auto-injector pen according to claim 10, wherein the activation sleeve (3) comprises a sleeve body (31) for protecting a needle; the sleeve body (31) is provided with symmetrical extension arms (32); and a tail end of each of the extension arms (32) is provided with an activation portion (33) for cooperative activation with the lugs (101) and a limiting portion (34) for cooperative limiting with the lugs (101).
